# FASCICULE DE BREVET EUROPEEN

(11) **EP 1 932 561 B1**
(45) Date de publication et mention de la délivrance du brevet: **17.02.2010**
(21) Numéro de dépôt: 07291546.5
(22) Date de dépôt: 18.12.2007
(51) Int. Cl.: A61N 1/05, A61B 5/04, A61N 1/36

(54) **Sonde à bras multiples et système pour neurostimulation électrique profonde comportant une telle sonde**
Sonde mit Mehrfachverzweigungen und System zur elektrischen Tiefen-Neurostimulation mit Hilfe einer solchen Sonde
Multiple-arm probe and system for deep brain stimulation including such a probe

(30) Priorité: 18.12.2006 FR 0611022
(43) Date de publication de la demande: 18.06.2008
(73) Titulaire: Commissariat à l'Energie Atomique, 75015 Paris (FR)
(72) Inventeur: Sauter-Starace, Fabien, 38170 Seyssinet-Pariset (FR); Benabid, Alim-Louis, 38240 Meylan (FR); Caillat, Patrice, 38000 Grenoble (FR)
(74) Mandataire: Priori, Enrico

(56) Documents cités:
- US-A1- 2002 062 143
- US-A1- 2002 116 042
- US-A1- 2004 199 235
- US-A1- 2005 075 681
- US-A1- 2005 171 558
- US-A1- 2006 122 677
- US-A1- 2006 129 203
- US-B1- 7 006 859

## Description

L'invention porte sur une sonde pour neurostimulation électrique profonde, et plus particulièrement pour électrostimulation cérébrale profonde. L'invention porte également sur un système pour neurostimulation électrique profonde comportant au moins une telle sonde.

La stimulation cérébrale profonde (« deep brain stimulation » en langue anglaise) est une technique thérapeutique comportant l'implantation d'un appareil médical connu comme stimulateur cérébral, qui envoie des impulsions électriques à des parties spécifiques du cerveau. Par exemple, la stimulation des nucléus de thalamus ou de l'hypothalamus peut être utilisée pour traiter des désordres moteurs tels que des tremblements, provoqués notamment par le syndrome de Parkinson (voir l'article de A. - L Benabid., P. Pollak, C. Gervason, D. Hoffmann, D.-M. Gao, M. Hommel, J.-E. Perret et J. de Rougemont : « Long-term suppression of tremor by chronic stimulation of the ventral intermediate thalamic nucleus. » The Lancet, Volume 337, N° 8738, 16 février 1991, Pages 403-406), et la stimulation du cortex cingulaire subgenual est utilisée à titre expérimental pour le traitement de formes particulièrement graves et résistantes au traitement de dépression clinique (H.S. Mayberg et al. « Deep Brain Stimulation for Treatment-Resistant Depression », Neuron, Vol. 45, pages 651-660, 3 mars 2005). Il est également envisagé de stimuler le nucleus hypothalamique postérieur pour traiter les céphalées en grappe, la matière grise périaqueducale pour atténuer la douleur et l'hypothalamus ventromédian pour soigner certains cas d'obésité (A.-L. Benabid, B. Wallace, J. Mitrofanis, C. Xia, B. Piallat, V. Fraix, A. Batir, P. Krack, P. Pollak et F. Berger, « Therapeutic electrical stimulation of the central nervous system » Comptes Rendus Biologies, Volume 328, N° 2, February 2005, Pages 177-186).

Dans tous les cas, une intervention de stimulation cérébrale profonde comporte l'insertion dans le crâne du patient d'une sonde souple, guidée par une canule et/ou un stylet rigide, jusqu'à que la pointe de ladite sonde atteigne la région du cerveau à stimuler. A proximité de sa pointe, la sonde comporte des électrodes (généralement quatre), qui sont reliés par un câble sous-cutané à un appareil de génération d'impulsions, implanté à son tour sous la peau du patient comme un stimulateur cardiaque conventionnel. La canule et/ou le stylet sont extraits du crâne du patient après avoir servi à l'introduction de la sonde, alors que cette dernière reste en place pendant une durée qui peut atteindre plusieurs années.

Une description plus détaillée de la procédure d'implantation d'une sonde pour stimulation cérébrale profonde est fournie par le document « Medtronic - DBS™ Lead Kit for Deep Brain Stimulation 3387 3389 - Implant manual » de la société Medtronic Inc., téléchargeable du site Internet : http:/lwww.medtronic.com/physician/activa/downloadablefiles/197928_b_006. pdf.

Des sondes pour neurostimulation électrique profonde de type conventionnel sont décrites, par exemple, dans le document précité de la société Medtronic, ainsi que dans le document US 6,512,958.

La forme rectiligne de la sonde est imposée par la nécessité de rendre son insertion la moins traumatique possible pour le patient. Cependant, elle a le défaut de ne permettre que la stimulation d'une très petite région de tissu cérébral, alors que, afin d'obtenir une meilleure efficacité du traitement, il serait souhaitable de pouvoir agir sur une cible de plus grand volume, disposer de plus de choix sur la ou les zones à stimuler sur une même trajectoire de la sonde ou pouvoir cerner spatialement une zone du cerveau du patient. L'implantation de plusieurs sondes, visant des points distincts d'une même région cible de volume relativement important, est possible, mais comporte une multiplication des risques et des effets collatéraux de l'intervention.

Le document EP1062973 décrit une sonde souple pour électrostimulation cérébrale profonde destinée à être introduite dans le cerveau d'un patient à l'aide d'une canule. L'extrémité de tête de cette sonde est constituée de quatre tiges pliables, chacune pourvue d'une électrode de stimulation et destinées à s'écarter les unes des autres lorsque ladite extrémité de tête sort de la canule. De cette manière, il est possible de stimuler un volume de tissu nerveux ayant une forme tétraédrique. L'inconvénient majeur de ce dispositif est que le mode de déploiement par des tiges pliables par écartement mutuel est susceptible de provoquer des lésions irréversibles importantes du tissu cérébral à stimuler.

Le document US2005/0267347 décrit une sonde de stimulation et mesure comportant une canule d'insertion rigide et un ou plusieurs électrodes flexibles destinées à être introduits dans le cerveau d'un patient par ladite canule d'insertion. L'extrémité de la canule présente une forme adaptée pour dévier ledit ou lesdits électrodes flexibles, de telle manière que la partie de ces électrodes qui dépasse de ladite extrémité présente un angle d'inclinaison par rapport à l'axe de cette dernière. Seulement un nombre limité d'électrodes peut être introduit à l'aide d'une même canule. De plus, le mécanisme de flexion des électrodes par l'extrémité de la canule ne permet pas d'orienter lesdites électrodes indépendamment les unes des autres. En outre, ce dispositif est destiné à être utilisé pour effectuer une étape d'exploration afin de déterminer le positionnement optimal d'une sonde de stimulation, mais ne convient pas en tant que sonde d'électrostimulation cérébrale profonde, car il ne peut pas être implanté à demeure.

Le document US 2005/075681 A1 décrit une sonde par neurostimulation à bras multiples selon le préambule de la revendication 1.

Un but de l'invention est donc de procurer une sonde pour neurostimulation électrique profonde permettant de stimuler une région de tissu nerveux plus important qu'une sonde rectiligne, ne présentant pas les inconvénients de l'art antérieur précité. En particulier, une sonde selon l'invention doit pouvoir être implantée à demeure et d'une manière relativement peu traumatique pour le patient.

Ce but est atteint, conformément à l'invention, par une sonde pour neurostimulation électrique profonde comportant : un corps tubulaire en matériau biocompatible, ayant une paroi latérale définissant une lumière, ledit corps tubulaire pouvant être introduit pour au moins une partie de sa longueur à l'intérieur du corps d'un patient pour atteindre une région à stimuler ; une pluralité de bras, portant chacun au moins une électrode de stimulation et pouvant passer d'une première position dans laquelle ils sont logés à l'intérieur dudit corps tubulaire à une deuxième position dans laquelle ils font saillie radialement à partir de la paroi latérale de ce dernier et inversement ; et des moyens pour faire passer lesdits bras de ladite première position à ladite deuxième position et inversement ; ladite sonde étant caractérisée en ce qu'une pluralité desdits bras sont reliés entre eux par un élément de base de forme tubulaire, disposé à l'intérieur de la lumière dudit corps tubulaire, et en ce que lesdits bras et ledit élément de base constituent une pièce unique en forme de feuillard, enroulée de manière à s'adapter à la forme de la lumière dudit corps tubulaire.

Selon des modes de réalisation particuliers de l'invention:
- Les bras faisant saillie de ladite paroi latérale à partir d'une même position axiale de cette dernière peuvent être reliés entre eux par un même élément de base de forme tubulaire, disposé à l'intérieur de la lumière dudit corps tubulaire.
Ledit feuillard peut présenter une épaisseur comprise entre 10 et 100 µm, et de préférence entre 12 et 50 µm.
- Une pluralité desdits bras peuvent faire saillie de ladite paroi latérale à partir d'une même position axiale de cette dernière.
- Ladite sonde peut comprendre une pluralité de groupes de bras, les bras de chaque groupe faisant saillie de ladite paroi latérale à partir d'une même position axiale de cette dernière et chacun desdits groupes correspondant à une position axiale différente.
- Lesdits bras peuvent être repartis régulièrement autour de la paroi latérale dudit corps tubulaire.
- Lesdits bras peuvent être logés au moins en partie dans des canaux s'étendant dans la paroi latérale dudit corps tubulaire, de préférence suivant une direction oblique par rapport à l'axe de ce dernier.
- Lesdits moyens pour faire passer lesdits bras de ladite première à ladite deuxième position peuvent comprendre un ressort agencé de manière à exercer sur lesdits bras une force dirigée parallèlement à l'axe dudit corps tubulaire, de manière à les pousser à l'extérieur de ce dernier à travers lesdits canaux.
- Lesdits bras peuvent être flexibles, et être agencés de manière à passer de ladite première à ladite deuxième position et inversement par un mouvement de translation.
- Selon une première variante, lesdits moyens pour faire passer lesdits bras de ladite deuxième à ladite première position peuvent comprendre une tige pouvant être actionnée en compression pour exercer une force opposée à celle dudit ressort, de manière à faire rentrer lesdits bras dans ledit corps tubulaire à travers lesdits canaux. En particulier, ladite tige peut être un stylet pouvant être introduit dans la lumière dudit corps tubulaire pour le rigidifier en vue de son insertion dans le corps d'un patient.
- Selon une première variante, les moyens pour faire passer lesdits bras de ladite deuxième à ladite première position peuvent comprendre un câble pouvant être actionné en traction pour exercer une force opposée à celle dudit ressort, de manière à faire rentrer lesdits bras dans ledit corps tubulaire à travers lesdits canaux.
- Une pluralité desdits bras peuvent être reliés entre eux par un élément de base de forme tubulaire, disposé à l'intérieur de la lumière dudit corps tubulaire.
- Lesdits bras peuvent être réalisés en un matériau électriquement isolant et présentent chacun au moins une électrode de stimulation sur sa surface respective. En particuliers, lesdits bras peuvent être réalisés, au moins en partie, en un matériau isolant biocompatible choisi parmi les polyimides, et en particulier les poly-isoindroquinazorindiones (PIQ) et le polyimide Pi2611, et les benzocyclobutènes (BCB).
- Au moins un desdits bras peut comporter également au moins une électrode de mesure, présentant une surface moindre de celle desdits électrodes de stimulation. En particulier, une pluralité d'électrodes de mesure peuvent être disposées à côté de chaque électrode de stimulation.
- Une telle sonde peut comprendre des éléments électriquement conducteurs s'étendant dans la lumière ou à l'intérieur de la paroi latérale dudit élément tubulaire et formant des contacts électriques avec les électrodes portées par lesdits bras.
- Les électrodes peuvent être des électrodes planaires, reliées auxdites éléments électriquement conducteurs par l'intermédiaire de pistes conductrices planaires formées sur lesdits bras.
- Lesdits éléments conducteurs peuvent s'étendre jusqu'à une extrémité dite postérieure dudit corps tubulaire et comprendre des moyens de raccordement à un générateur d'impulsions électriques pour neurostimulation électrique profonde.
- Ledit corps tubulaire peut être réalisé en un matériau biocompatible choisi parmi les silicones, les siloxanes, le polyuréthane et le polyvinyle chlorure. Il peut présenter une forme sensiblement cylindrique avec un diamètre compris entre 500 µm et 5 mm, et de préférence entre 500 µm et 2 mm.

Le nombre total de bras peut être compris entre 3 et 60, et de préférence entre 4 et 40.
- Lesdits bras peuvent présenter une longueur comprise entre 0,2 et 3 mm, et de préférence entre 0,4 et 2 mm.
- La longueur du corps tubulaire de la sonde peut être comprise entre 15 cm et 40 cm.

Un autre objet de l'invention est un système de neurostimulation électrique profonde comportant un générateur d'impulsions électriques pour neurostimulation électrique profonde; et au moins une sonde telle que décrite ci-dessus, dont les électrodes de stimulation sont raccordées électriquement audit générateur d'impulsions électriques.

Lorsqu'au moins un bras de ladite sonde comporte au moins une électrode de mesure, ledit système peut comporter également un instrument de mesure raccordé électriquement auxdites électrodes de mesure.

D'autres caractéristiques, détails et avantages de l'invention ressortiront à la lecture de la description faite en référence aux dessins annexés donnés à titre d'exemple et qui représentent, respectivement :
- La figure 1, une vue en coupe longitudinale de l'extrémité antérieure d'une sonde pour neurostimulation électrique profonde selon un premier mode de réalisation de l'invention, dans sa position repliée d'insertion ;
- La figure 2, une vue en coupe longitudinale de l'extrémité antérieure de cette même sonde dans sa position dépliée ;
- La figure 3, une vue en coupe transversale le long de la ligne III-III de cette même sonde en position repliée ;
- La figure 4, la pièce en forme de feuillard constituant les bras dépliables de la sonde, montrée à l'état déroulé ;
- La figure 5, un vue agrandie de l'un des bras de la sonde ;
- La figure 6, une vue en coupe longitudinale de l'extrémité antérieure d'une sonde pour neurostimulation électrique profonde selon un deuxième mode de réalisation de l'invention, dans sa position repliée d'insertion ; et
- La figure 7, un schéma de principe d'un Système de neurostimulation électrique profonde comprenant une sonde selon ledit premier mode de réalisation de l'invention, introduite dans le troisième ventricule du cerveau d'un patient.

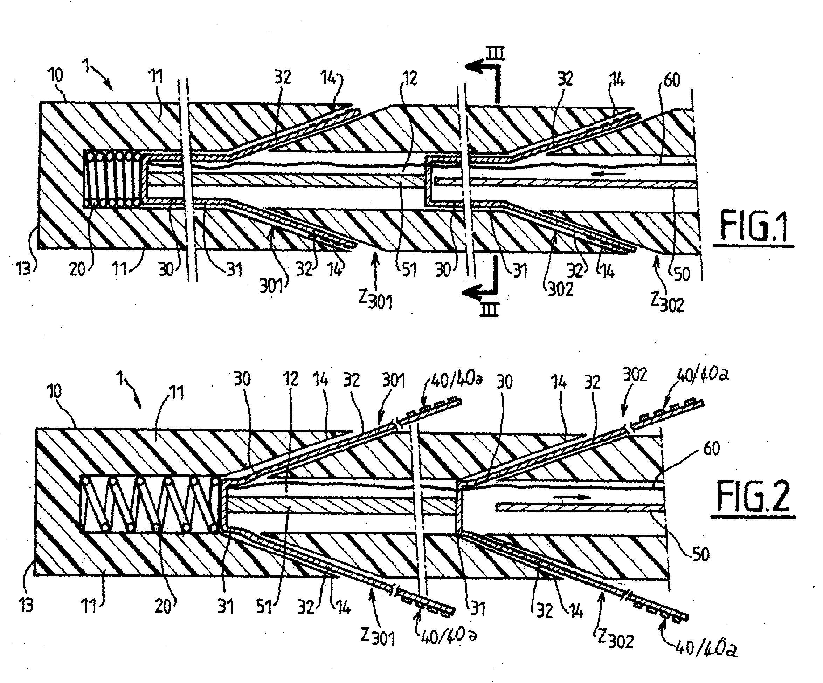

Comme illustré par les figures 1 et 2, une sonde 1 selon un premier mode de réalisation de l'invention comporte un corps tubulaire 10 réalisé en matériau biocompatible souple, ayant une paroi latérale 10 définissant une lumière 12 et une extrémité antérieure fermée 13, formant une pointe par laquelle la sonde peut être introduite dans le corps d'un patient, par exemple dans son cerveau. Typiquement, la sonde 1 peut être fabriquée par moulage, un insert étant utilisé pour obtenir une forme tubulaire.

Le corps tubulaire 10 présente une forme sensiblement cylindrique, avec un diamètre compris entre 0,5 et 5 mm environ, et de préférence entre 0,5 et 2 mm, de manière que son insertion dans un cerveau humain (ou, le cas échéant, dans un autre tissu nerveux tel que la moelle épinière), ne provoque pas de lésions graves ; sa longueur est typiquement de l'ordre de 15 cm à 40 cm selon les protocoles d'implantation.

Tout matériau biocompatible souple peut être utilisé pour la fabrication d'une sonde selon l'invention. En particulier, on peut citer les silicones, les siloxanes, le polyuréthane et le polyvinyle chlorure.

Un ressort 20 est logé dans la lumière 12 du corps tubulaire 10, prenant appui sur le côté intérieur de l'extrémité fermée 13 de ce dernier ; on comprend que le matériau constituant le corps tubulaire, bien que généralement défini comme « souple », 10 devra présenter une rigidité suffisante pour fournir un appui suffisant au ressort 20. Le ressort 20 est représenté dans sa position comprimée sur la figure 1 et dans sa position détendue sur la figure 2. Bien que les figures 1 et 2 montrent un ressort à spirale, cela ne constitue nullement une limitation. En variante il sera possible d'utiliser, par exemple, un ressort à lame ou même un simple bloc de mousse ou de matériau élastomère. Le ressort 20 pourra être constitué de métal, par exemple d'acier inoxydable à haute limite d'élasticité de type XC80, d'un polymère plus souple axialement que le corps tubulaire 10, voire d'un polymère photosensible. Dans ce dernier cas, le ressort 20 pourra être fabriqué par photolithographie ou par prototypage rapide.

Deux pièces 30 sont logées de manière coulissante à l'intérieur de la lumière 12 du corps tubulaire 10, de manière à pouvoir être déplacée par le ressort 20 lorsque ce dernier passe de sa position comprimée (figure 1) à sa position détendue (figure 2). Chaque pièce 30 comporte un élément de base 31 de forme sensiblement cylindrique, pleine ou creuse, et d'une pluralité de bras 32 s'étendant dans une direction axiale opposée au ressort 20 à partir de la périphérie dudit élément de base 31.

Les deux pièces 30 sont reliées entre elles par un insert rigide 51 permettant la transmission d'un mouvement de déplacement axial à l'intérieur de la lumière 12 du corps tubulaire 10.

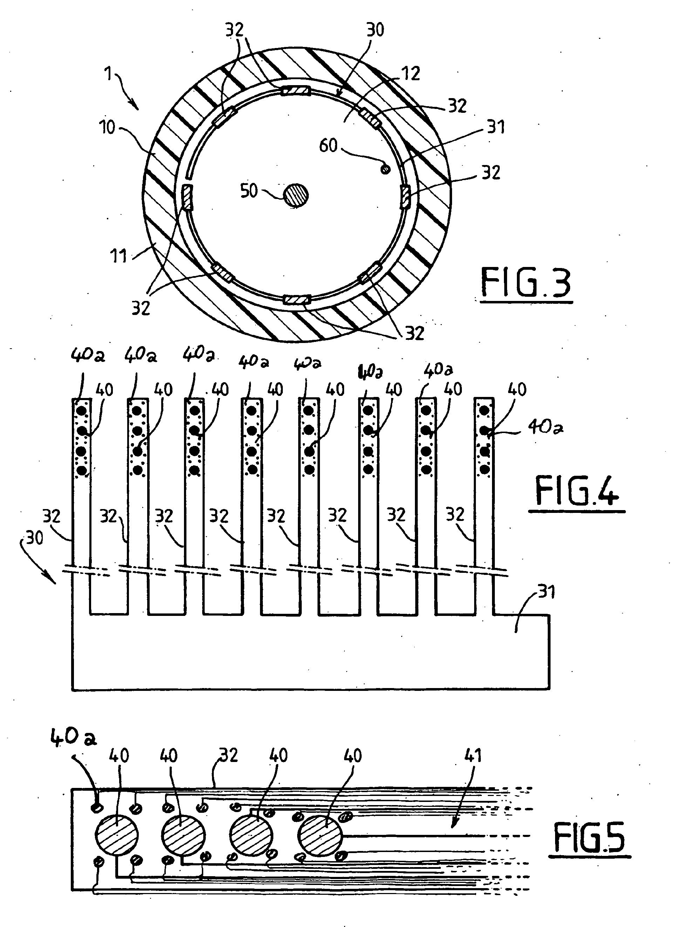

Comme représenté sur les figures 3 et 4, chaque pièce 30 est constituée par un feuillard découpé de manière à former une pluralité de bras de forme allongée 32, relies entre eux par l'élément de base 31 en forme de bande et s'étendant perpendiculairement à ce dernier. Le feuillard, d'une épaisseur comprise typiquement entre 12 µm et 50 µm, est enroulé pour être introduit dans la lumière 12 du corps tubulaire souple 10, comme illustré par la figure 3. Les figures 3 et 4 montrent une pièce comportant huit bras, mais il ne s'agit que d'un exemple ; le nombre de bras 32 de chaque pièce 30 peut être typiquement compris entre 3 et 12, et de préférence entre 4 et 8, et le nombre de pièces 30 peut être compris entre 1 et 5.

Chacune des pièces 30 forme un groupe de bras, indiqués par les chiffres de référence 301 et 302. Les bras 32 appartenant à un même groupe font saillie de la paroi latérale 11 du corps tubulaire 10 en correspondance d'une même position axiale Z₃₀₁, Z₃₀₂ ; chaque groupe de bras correspond à une position axiale différente. De cette manière, lorsque les bras 32 se trouvent dans leur position dépliée, la sonde 1 selon l'invention prend une configuration qui rappelle celle d'un parapluie (si un seul groupe de bras est présent) ou d'un sapin (si plusieurs groupes sont prévus). De préférence, les bras 32 sont repartis régulièrement autour de la paroi latérale 11 du corps tubulaire 10.

Chacun des bras 32 présente des électrodes planaires de stimulation 40, typiquement au nombre de quatre, qui servent pour appliquer des impulsions électriques au tissu cérébral cible générées par un appareil de stimulation. Des électrodes de mesure 40a, ayant de plus petites surfaces que les électrodes de stimulation 40, peuvent également être prévus à proximité de ces derniers. Typiquement, on peut prévoir quatre électrodes de mesure entourant chaque électrode de stimulation.

La disposition d'électrodes d'enregistrement à proximité des électrodes de stimulation sert à assurer une bonne localisation de ces dernières. Dans le cas particulier de la maladie de Parkinson, la cible du noyau subthalamique est identifiée d'une part par imagerie X et IRM et d'autre part par des enregistrements électrophysiologiques de la zone à stimuler. La sonde décrite par la présente invention permet de positionner la sonde par les procédés d'imagerie, de déployer les bras de la sonde et d'effectuer dans cette position la tâche d'enregistrement électrophysiologique afin de localiser plus finement la meilleure électrode de stimulation.

Pour d'autres indications telles que l'épilepsie, ces électrodes d'enregistrement permettent de détecter la survenue d'une crise. Le générateur d'impulsions peut alors alimenter les électrodes de stimulation avec un stimulus capable de stopper la propagation de la crise.

Ces électrodes 40, ainsi que les pistes planaires 41 amenant à ces dernières lesdites impulsions électriques, peuvent être réalisés par les techniques conventionnelles des semi-conducteurs ou circuits imprimés, par exemple par un procédé de dépôt par pulvérisation ou évaporation ou dépôt électrolytique (dépôt chimique - CVD - ou physique - PVD - en phase vapeur) pour les couches conductrices suivi par centrifugation (spin coating) lithographie et gravure physique ou chimique. D'autres techniques susceptibles d'être utilisées pour réaliser lesdites pistes planaires comprennent l'utilisation d'encres conductrices ou de poudres compactées par frittage, notamment par frittage laser, ainsi que le dépôt pleine plaque suivi par une ablation laser.

Le feuillard à partir duquel la pièce 30 est réalisée doit être constitué d'un matériau électriquement isolant, biocompatible et suffisamment flexible pour permettre son enroulement. Par exemple, il peut s'agir d'un matériau choisi parmi les polyimides, et en particulier les poly-isoindroquinazorindiones (PIQ) et le polyimide Pi2611, et les benzocyclobutène. Les polyimides sont particulièrement adaptés car ils se prêtent bien à la photolithographie et sont couramment utilisés en électronique. Des procédés de libération connus de l'art antérieur permettent notamment la création d'un assemblage souple constitué d'une couche conductrice et de deux couches en polymère isolant en utilisant des couches d'accroche chome-aluminium, la dissolution anodique de l'aluminium permettant de désolidariser l'empilement du substrat. Ces procédés sont décrits en particulier dans l'article de Leonardi, P. Leunberger, D. Bertrand, S. Nielsen, A. Bertsch et Ph. Renaud, « Novel Noninvasive Method for Intraocular Pressure Monitoring using Pressure Sensing Contact Lens », European Glaucoma Society '04, Florence, 30 mai - 4 juin, 2004.

Les impulsions électriques de neurostimulation sont amenées aux pistes conductrices 41 des bras 32 par des éléments conducteurs 60, tels que des câbles métalliques, s'étendant dans la lumière 12 du corps tubulaire 10 ou à l'intérieur de sa paroi latérale 11, jusqu'à son extrémité postérieure ouverte.

Les différents éléments conducteurs de la sonde 1 peuvent être réalisés en métal, en ITO (oxyde d'indium et aluminium) ou graphite. Les éléments conducteurs destinés à être en contact avec les tissus corporels du patient, tel que les électrodes 40, devront bien entendu être réalisés en un matériau biocompatible, tel que le platine.

Les dimensions des électrodes 40 sont déterminées en fonction du courant de stimulation prévu, sachant qu'il n'est pas souhaitable de dépasser une densité de courant de 1 mA/mm², par analogie avec les valeurs utilisées couramment en électrostimulation cérébrale profonde avec des sondes de type conventionnel. Les électrodes de mesure 40a peuvent avoir, typiquement, une surface comprise entre 400 µm² et 6 mm².

Les bras 32, d'une longueur typiquement comprise entre 0,2 et 3 mm, sont entièrement logés à l'intérieur du corps tubulaire 10 lorsque le ressort 20 se trouve à l'état comprimé (première position, figure 1). Plus précisément, les bras 32 s'étendent pour la plupart de leur longueur à l'intérieur de la lumière 12, mais leurs extrémités sont logées dans des canaux obliques 14, orientés dans une direction opposée à celle de la pointe 13 de la sonde. Avantageusement, les canaux 14 forment un angle compris entre 10° et 90°, et de préférence entre 20° et 45° avec l'axe du corps tubulaire 10.

De cette manière, lorsque le ressort se détend (deuxième position, figure 2) il pousse les bras 32 vers l'extérieur de la sonde 1 à travers lesdits canaux obliques 14. Ainsi, les électrodes 40 disposés à leurs extrémités peuvent contacter des régions de tissu cérébral relativement éloignées de l'axe de la sonde et stimuler une région cible de volume relativement grand.

La réversibilité du mouvement est importante pour permettre l'extraction de la sonde 1 sans léser plus de tissus que ne le ferait la sonde 1 seule.

Comme les bras 32 ne dépassent pas du périmètre du corps tubulaire 10 lors de l'insertion et de l'extraction de la sonde, ces opérations ne sont pas plus traumatiques pour le patient que si une sonde conventionnelle avait été utilisée. Cependant, le volume de la région stimulée peut être beaucoup plus important.

L'actionnement du ressort 20 pour faire passer les bras 32 de leur première à leur deuxième position et inversement est rendu possible par un stylet rigide 50 traversant la lumière 12 du corps tubulaire. Un tel stylet, réalisé par exemple en métal, est généralement prévu dans les sondes pour neurostimulation électrique connues de l'art antérieur afin de rigidifier le corps tubulaire souple de manière à permettre son insertion. Dans le cas de l'invention, lorsque la sonde 1 est introduite dans le crâne du patient, le stylet 50 comprime le ressort 20 et maintient les bras 32 dans leur première position repliée. Une fois que la sonde 1 a atteint son emplacement définitif, le stylet 50 est extrait, ce qui permet au ressort 50 de se détendre et faire passer les bras 32 dans leur deuxième position dépliée. Pour permettre une extraction sans danger de la sonde 1, il est nécessaire d'introduire à nouveau le stylet 50 dans le corps tubulaire 11 afin de replier à nouveau les bras 32.

Globalement, les bras 32 accomplissent un mouvement de translation pure pour passer de leur première à leur deuxième position et inversement, ce qui permet de minimiser l'endommagement du tissu à stimuler. Il s'agit là d'un avantage majeur de l'invention par rapport au dispositif du document EP1062973 précité. Ce mouvement de translation se fait le long d'une trajectoire comportant un coude à la transition entre la lumière 12 et les canaux 14. On comprend que les bras doivent être suffisamment souples pour pouvoir fléchir au niveau de ce coude, et en même temps suffisamment rigides pour pouvoir surmonter la résistance opposée à leur déploiement par le tissu cible. Cependant, dans certains cas il sera possible d'effectuer ce déploiement dans une cavité remplie de liquide céphalorachidien, par exemple le troisième ventricule qui est limitrophe au thalamus et à l'hypothalamus, qui sont des régions cible traditionnelles pour l'électrostimulation cérébrale.

Pour permettre l'assemblage des différents éléments de la sonde 1, et en particulier l'introduction du ressort 20 et les pièces coulissantes 30 dans la lumière 12 du corps tubulaire 10, il est préférable que l'extrémité fermée 13 de ladite sonde 1 soit constituée par un bouchon amovible.

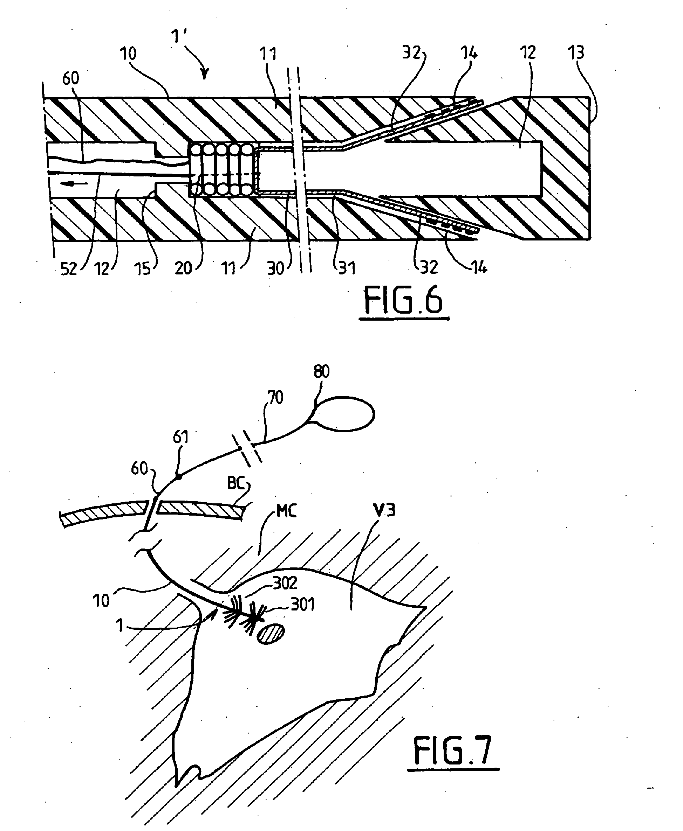

La figure 6 montre une sonde 1' selon un deuxième mode de réalisation de l'invention. Dans la sonde 1', les canaux 14 et les bras 32 sont orientés vers l'extrémité antérieure fermée 13 du corps tubulaire 10. Le ressort 20 ne prend donc pas appui sur cette extrémité antérieure, mais sur un rétrécissement de la lumière ou marche 15. L'actionnement du ressort 20 n'est pas obtenu à l'aide d'un stylet d'insertion, mais d'un câble 52 fixé à la pièce coulissante 30 : une force de traction exercée sur ledit câble 52 permet de comprimer le ressort 20 et de maintenir les bras 32 dans leur première position repliée, tandis que le relâchement dudit câble permet au ressort 20 de se détendre et amener lesdits bras 32 dans leur deuxième position dépliée. Dans le cas de ce deuxième mode de réalisation de l'invention, l'insertion de la sonde 1' pourra être effectuée à l'aide d'une canule plutôt que d'un stylet. L'avantage de ce deuxième mode de réalisation de l'invention est que l'extraction de la sonde est beaucoup moins traumatique que dans le cas du premier mode de réalisation, en cas de défaillance empêchant de ramener les bras 32 dans leur position repliée.

Bien entendu, dans le cas de ce mode de réalisation il sera nécessaire de veiller à que la mise en tension du câble 52 pendant l'introduction de la sonde ne provoque pas son flambement. Pour ce faire, il est possible d'utiliser un stylet de rigidification passant à travers la pièce coulissant 30 et le ressort 20 pour s'appuyer directement sur l'extrémité fermée 13 de la sonde. Alternativement, il est possible d'effectuer l'insertion à l'aide d'une canule rigide.

La figure 7 montre de manière schématique un système d'électrostimulation cérébrale utilisant une sonde 1 selon le premier mode de réalisation de l'invention, représentée à l'état déployé à l'intérieur du troisième ventricule V3 du cerveau d'un patient. D'une manière connue en soi, le corps tubulaire 10 s'étend à travers la matière cérébrale MC jusqu'à ressortir du crâne du patient par l'intermédiaire d'un orifice pratiqué dans la boîte crânienne BC de ce dernier. En correspondance de l'extrémité postérieure du corps tubulaire 10, les éléments conducteurs 60 sont reliés par l'intermédiaire de moyens de raccordement 61 à un câble sous-cutané qui est à son tour relié à un générateur d'impulsions électrique pour neurostimulation profonde 80.

Si la sonde 1 comporte également des électrodes de mesure, le dispositif implanté 80 pourra comprendre également un instrument de mesure raccordé électriquement à ces dernières. En variante, les électrodes de mesure peuvent être utilisées uniquement lors de la phase d'implantation de la sonde (utilisation peropératoire) : dans ce cas il peut être inutile de prévoir un instrument de mesure implanté.

L'invention a été décrite en référence à un mode de réalisation particulier, destiné à l'électrostimulation cérébrale profonde ; cependant, l'invention peut s'appliquer plus généralement à la neurostimulation électrique profonde d'autres tissus, tels que la moelle épinière.

Diverses variantes structurelles sont également envisageables.

Par exemple, l'actionnement des bras 32 peut être obtenu par des moyens autres qu'un ressort, par exemple un métal à mémoire de forme ou un actionneur piézoélectrique. Dans ces cas, l'utilisation d'un stylet peut ne pas être essentielle, et l'insertion être effectuée à l'aide d'une canule rigide.

Les bras peuvent comporter plus ou moins de quatre électrodes ; la partie d'extrémité antérieure du corps tubulaire 10 peut porter, lui aussi, des électrodes, comme dans une sonde conventionnelle.

Enfin, des matériaux différents de ceux décrits explicitement peuvent être utilisés pour la réalisation des différents composants de l'invention, à la condition qu'ils possèdent des propriétés mécaniques et de biocompatibilité adaptées, ce qui pourra être déterminé sans difficulté par l'homme du métier.

## Revendications

1. Sonde (1, 1') pour neurostimulation électrique profonde comportant :
- un corps tubulaire (10) en matériau biocompatible, ayant une paroi latérale (11) définissant une lumière (12), ledit corps tubulaire (10) pouvant être introduit pour au moins une partie de sa longueur à l'intérieur du corps d'un patient pour atteindre une région à stimuler ;
- une pluralité de bras (32), portant chacun au moins une électrode de stimulation (40) et pouvant passer d'une première position dans laquelle ils sont logés à l'intérieur dudit corps tubulaire (11) à une deuxième position dans laquelle ils font saillie radialement à partir de la paroi latérale (11) de ce dernier et inversement ; et
- des moyens (20, 50) pour faire passer lesdits bras (32) de ladite première position à ladite deuxième position et inversement ;
**caractérisée en ce que**
- une pluralité desdits bras (32) sont reliés entre eux par un élément de base (31) de forme tubulaire, monté de manière coulissante à l'intérieur de la lumière (12) dudit corps tubulaire (10) ; et **en ce que**
- lesdits bras (32) et ledit élément de base (31) constituent une pièce unique (30) en forme de feuillard, enroulée de manière à s'adapter à la forme de la lumière (12) dudit corps tubulaire (10).

2. Sonde (1, 1') selon la revendication 1, dans laquelle tous les bras (32) faisant saillie de ladite paroi latérale (11) à partir d'une même position axiale (Z₃₀₁, Z₃₀₂) de cette dernière sont reliés entre eux par un même élément de base (31) de forme tubulaire, disposé à l'intérieur de la lumière dudit corps tubulaire (10).

3. Sonde (1, 1') selon la revendication 2, dans laquelle ledit feuillard présente une épaisseur comprise entre 10 et 100 µm, et de préférence entre12 µm et 50 µm.

4. Sonde (1, 1') selon l'une des revendications précédentes, dans laquelle une pluralité desdits bras (32) font saillie de ladite paroi latérale (11) à partir d'une même position axiale (Z₃₀₁, Z₃₀₂) de cette dernière

5. Sonde (1) selon la revendication 4, comprenant une pluralité de groupes de bras (301, 302), les bras (32) de chaque groupe (301, 302) faisant saillie de ladite paroi latérale (11) à partir d'une même position axiale (Z₃₀₁, Z₃₀₂) de cette dernière et chacun desdits groupes (301, 302) correspondant à une position axiale différente (Z₃₀1, Z₃₀₂).

6. Sonde (1, 1') selon l'une des revendications précédentes, dans laquelle lesdits bras (32) sont repartis régulièrement autour de la paroi latérale (11) dudit corps tubulaire (10).

7. Sonde (1, 1') selon l'une des revendications précédentes, dans laquelle les bras (32) sont logés au moins en partie dans des canaux (14) s'étendant dans la paroi latérale (11) dudit corps tubulaire (10).

8. Sonde (1, 1') selon la revendication 7, dans laquelle les bras (32) s'étendent dans la paroi latérale (11) dudit corps tubulaire (10) suivant une direction oblique par rapport à l'axe de ce dernier.

9. Sonde (1, 1') selon la revendication 7 ou 8, dans laquelle les moyens pour faire passer lesdits bras de ladite première à ladite deuxième position comprennent un ressort (20) agencé de manière à exercer sur lesdits bras (32) une force dirigée parallèlement à l'axe dudit corps tubulaire (11), de manière à les pousser à l'extérieur de ce dernier à travers lesdits canaux (14).

10. Sonde (1, 1') selon la revendication 9, dans laquelle lesdits bras (32) sont flexibles, et sont agencés de manière à passer de ladite première à ladite deuxième position et inversement par un mouvement de translation.

11. Sonde (1) selon l'une des revendications 9 ou 10, dans laquelle les moyens pour faire passer lesdits bras de ladite deuxième à ladite première position comprennent une tige (50) pouvant être actionnée en compression pour exercer une force opposée à celle dudit ressort (20), de manière à faire rentrer lesdits bras dans ledit corps tubulaire (11) à travers lesdits canaux (14).

12. Sonde (1) selon la revendication 11, dans laquelle ladite tige est un stylet (50) pouvant être introduit dans la lumière (12) dudit corps tubulaire (11) pour le rigidifier en vue de son insertion dans le corps d'un patient.

13. Sonde (1') selon l'une des revendication 9 ou 10, dans laquelle les moyens pour faire passer lesdits bras de ladite deuxième à ladite première position comprennent un câble (52) pouvant être actionné en traction pour exercer une force opposée à celle dudit ressort (20), de manière à faire rentrer lesdits bras dans ledit corps tubulaire (11) à travers lesdits canaux (14).

14. Sonde (1, 1') selon l'une des revendications précédentes, dans laquelle lesdits bras (32) sont réalisés en un matériau électriquement isolant et présentent chacun au moins une électrode (40) sur sa surface respective.

15. Sonde (1, 1') selon la revendication 14, dans laquelle lesdits bras (32) sont réalisés, au moins en partie, en un matériau isolant biocompatible choisi parmi les polyimides, et en particulier les polyisoindroquinazorindiones (PIQ) et le polyimide Pi2611, et les benzocyclobutènes.

16. Sonde selon l'une des revendications précédentes, dans laquelle au moins un desdits bras (32) comporte également au moins une électrode de mesure (40a), présentant une surface moindre de celle desdits électrodes de stimulation (40).

17. Sonde selon la revendication 16 dans laquelle une pluralité d'électrodes de mesure (40a) sont disposées à côté de chaque électrode de stimulation.

18. Sonde (1, 1') selon l'une des revendications précédentes, comprenant des éléments électriquement conducteurs (60) s'étendant dans la lumière ou à l'intérieur de la paroi latérale dudit élément tubulaire et formant des contacts électriques avec les électrodes (40, 40a) portées par lesdits bras.

19. Sonde (1, 1') selon la revendication 18, dans laquelle lesdites électrodes (40, 40a) sont des électrodes planaires, reliées auxdites éléments électriquement conducteurs (60) par l'intermédiaire de pistes conductrices planaires (41) formées sur lesdits bras (32).

20. Sonde (1, 1') selon l'une des revendications 18 ou 19, dans laquelle lesdits éléments conducteurs (60) s'étendent jusqu'à une extrémité dite postérieure dudit corps tubulaire (10) et comprennent des moyens de raccordement (61) à un générateur d'impulsions électriques pour neurostimulation électrique profonde (80).

21. Sonde (1, 1') selon l'une des revendications précédentes, dans laquelle ledit corps tubulaire (10) est réalisé en un matériau biocompatible choisi parmi les silicones, les siloxanes, le polyuréthane et le polyvinyle chlorure.

22. Sonde (1, 1') selon l'une des revendications précédentes, dans laquelle ledit corps tubulaire (10) présente une forme sensiblement cylindrique avec un diamètre compris entre 0,5 et 5 mm, et de préférence entre 0,5 et 2 mm.

23. Sonde (1, 1') selon l'une des revendications précédentes, comprenant un nombre de bras (32) compris entre 3 et 60, et de préférence entre 4 et 40.

24. Sonde (1, 1') selon l'une des revendications précédentes, dans laquelle lesdits bras (32) présentent une longueur comprise entre 0,2 et 3 mm, et de préférence entre 0,4 et 2 mm.

25. Sonde selon l'une des revendications précédentes, ayant un corps tubulaire (10) d'une longueur comprise entre 15 cm et 40 cm.

26. Système de neurostimulation électrique profonde comportant :
- un générateur d'impulsions électriques pour neurostimulation électrique profonde (80) ; et
- au moins une sonde (1) selon l'une des revendications précédentes, dont les électrodes de stimulation sont raccordées électriquement audit générateur d'impulsions électriques (80).

27. Système de neurostimulation électrique selon la revendication 26, dans lequel au moins un bras (32) de ladite sonde comporte au moins une électrode de mesure (40a), présentant une surface moindre de celle desdits électrodes de stimulation (40), ledit système comportant également un instrument de mesure (80) raccordé électriquement auxdites électrodes de mesure (40a).

## Claims

1. A probe (1, 1') for deep electrical neurostimulation comprising:
- a tubular body (10) of biocompatible material with a lateral wall (11) defining a lumen (12), said tubular body (10) can be introduced for at least a part of its length inside a patient's body for reaching a region to be stimulated;
- a plurality of arms (32) each bearing at least one stimulation electrode (40) and being able to pass from a first position in which they are housed inside of said tubular body (11) to a second position in which they project radially from the lateral wall (11) of this latter and inversely; and
- means (20 and 50) for making said arms (32) pass from said first position to said second position and inversely;
**characterized in that** a plurality of said arms (32) are connected together by a base element (31) of tubular shape placed inside of the lumen (12) of said tubular body (10); and **in that**
said arms (32) and said base element (31) make up a single part (30) of strap shape, wound so as to adapt to the shape of the lumen (12) of said tubular body (10).

2. The probe (1, 1') of claim 1, in which all the arms (32) projecting from said lateral wall (11) from a single axial position (Z₃₀₁ and Z₃₀₂) of this latter are connected together by a single tubular-shaped base element (31) placed inside the lumen of said tubular body (10).

3. The probe (1, 1') of claim 2, in which said strap has a thickness between 10 and 100 µm, and preferably between 12 and 50 µm.

4. The probe (1, 1') of any preceding claim, in which a plurality of said arms (32) project from said lateral wall (11) from a single axial position (Z₃₀₁, Z₃₀₂) of this latter.

5. The probe (1) of claim 4 including a plurality of groups of arms (301 and 302), the arms (32) of each group (301 and 302) projecting from said lateral wall (11) from a single axial position (Z₃₀₁ and Z₃₀₂) of this latter and each of said groups (301, 302) corresponding to a different axial position (Z₃₀₁ and Z₃₀₂).

6. The probe (1, 1') of any preceding claim, in which said arms (32) are evenly distributed around the lateral wall (11) of said tubular body (10).

7. The probe (1, 1') of any preceding claim, in which the arms (32) are housed at least in part in the channels (14) extending in the lateral wall (11) of said tubular body (10).

8. The probe (1, 1') of claim 7, in which the arms (32) extend in the lateral wall (11) of said tubular body (10) along an oblique direction relative to the axis of this latter.

9. The probe (1, 1') of claim 7 or 8, in which the means for making said arms pass from said first to said second position include a spring (20) arranged so as to exert on said arms (32) a force directed parallel to the axis of said tubular body (11), so as to push them to the outside of this latter through said channels (14).

10. The probe (1, 1') of claim 9, in which said arms (32) are flexible, and are arranged so as to pass from said first to said second position and inversely by translational movement.

11. The probe (1) of any preceding claim 9 or 10, in which the means to make said arms pass from said second to said first position include a stem (50) which can be actuated in compression for exerting a force opposed to that of said spring (20) so as to make said arms return into said tubular body (11) through said channels (14).

12. The probe (1) of claim 11, in which said stem is a stylus (50) which can be introduced in the lumen (12) of said tubular body (11) to make it rigid in order for its insertion in a patient's body.

13. The probe (1') of any preceding claim 9 or 10, in which the means to make said arms pass from said second to said first position include a cable (52) which can be actuated in traction for exerting a force opposed to that of said spring (20) so as to make said arms return into said tubular body (11) through said channels (14).

14. The probe (1, 1') of any preceding claim, in which said arms (32) are made of an electrically insulating material and each have at least one electrode (40) on its respective surface.

15. The probe (1, 1) of claim 14, in which said arms (32) are made, at least in part, from a biocompatible insulating material chosen from among the polyimides, and in particular polyisoindroquinazorindiones (PIQ) and the polyimide Pi2611, and the benzocyclobutenes (BCB).

16. The probe of any preceding claim, wherein at least one of said arms (32) also comprises at least a measurement electrode (40a) having a smaller surface than that of said stimulation electrod (40).

17. The probe of claim 16, wherein a plurality of measurement electrodes (40a) are disposed near to each stimulation electrode.

18. The probe (1, 1') of any preceding claim, including electrically conducting elements (60) extending into the lumen or inside of the lateral wall of said tubular element and forming electrical contacts with the electrodes (40, 40a) carried by said arms.

19. The probe (1, 1') of claim 18, in which said electrodes (40, 40a) are planar electrodes connected to said electrically conducting elements (60) by means of planar conducting tracks (41) formed on said arms (32).

20. The probe (1, 1') of any preceding claim 18 or 19, in which said conducting elements (60) extend to an end referred to as posterior of said tubular body (10) and include means of connection (61) to an electrical pulse generator for deep electrical neurostimulation (80).

21. The probe (1, 1') of any preceding claim, in which said tubular body (10) is made of a biocompatible material chosen from among the silicones, siloxanes, polyurethane and polyvinyl chloride.

22. The probe (1, 1') of any preceding claim, in which said tubular body (10) has a substantially cylindrical shape with a diameter between 0.5 and 5 mm and preferably between 0.5 and 2 mm.

23. The probe (1, 1') of any preceding claim, including a number of arms (32) between 3 and 60 and preferably between 4 and 40.

24. The probe (1, 1') of any preceding claim, in which said arms (32) have a length between 0.2 and 3 mm, and preferably between 0.4 and 2 mm.

25. The probe of any preceding claim, having a tubular body (10) with a length between 15 cm and 40 cm.

26. A system for deep electrical neurostimulation comprising:
- an electrical pulse generator for deep electrical neurostimulation (80); and
- at least one probe (1) as claimed in one of the preceding claims, whose stimulation electrodes are electrically connected to said electrical pulse generator (80).

27. The system of claim 26 wherein at least an arm (32) of said probe comprises at least a measurement electrode (40a) having a smaller surface than that of said stimulation electrodes (40), said system also comprising a measurement instrument (80) electrically connected to said measurement electrodes (40a).

## Patentansprüche

1. Sonde (1, 1') zur elektrischen Tiefenneurostimulation, umfassend:
- einen schlauchförmigen Körper (10) aus biokompatiblem Material, der eine Seitenwand (11) hat, die ein Lumen (12) definiert, wobei der schlauchförmige Körper (10) mit wenigstens einem Teil seiner Länge in das Innere des Körpers eines Patienten eingeführt werden kann, um eine zu stimulierende Region zu erreichen;
- eine Vielzahl von Armen (32), die jeweils wenigstens eine Stimulationselektrode (40) tragen und von einer ersten Position, in der sie im Inneren des schlauchförmigen Körpers (11) untergebracht sind, in eine zweite Position, in der sie radial aus der Seitenwand (11) dieses Letztgenannten vorstehen, übergehen können und umgekehrt, und
- Mittel (20, 50), um zu bewirken, dass die Arme (32) von der ersten Position in die zweite Position übergehen und umgekehrt,
**dadurch gekennzeichnet, dass**
- eine Vielzahl der Arme (32) untereinander durch ein Basiselement (31) mit röhrenförmiger Form verbunden sind, das in verschiebbarer Art im Inneren des Lumens (12) des schlauchförmigen Körpers (10) montiert ist, und dadurch, dass
- die Arme (32) und das Basiselement (31) ein einziges Stück (30) in Form eines Bandes bilden, dass so aufgerollt ist, dass es sich an die Form des Lumens (12) des schlauchförmigen Körpers (10) anpasst.

2. Sonde (1, 1') gemäß Anspruch 1, in der alle Arme (32), die aus der Seitenwand (11) ausgehend von derselben axialen Position (Z₃₀₁, Z₃₀₂) dieser Letztgenannten vorstehen gelassen werden, untereinander durch ein selbes Basiselement (31) mit röhrenförmiger Form, das im Inneren des Lumens des schlauchförmigen Körpers (10) angeordnet ist, verbunden sind.

3. Sonde (1, 1') gemäß Anspruch 2, in der das Band eine Dicke zwischen 10 und 100 µm und vorzugsweise zwischen 12 µm und 50 µm aufweist.

4. Sonde (1, 1') gemäß einem der vorangehenden Ansprüche, in der eine Vielzahl der Arme (32) ausgehend von derselben axialen Position (Z3₀₁, Z₃₀₂) dieser Letztgenannten vorstehen gelassen wird.

5. Sonde (1) gemäß Anspruch 4, die eine Vielzahl von Gruppen von Armen (301, 302) umfasst, wobei die Arme (32) jeder Gruppe (301, 302) aus der Seitenwand (11) ausgehend von derselben axialen Position (Z₃₀₁, Z₃₀₂) der Letztgenannten vorstehen gelassen werden und jede der Gruppen (301, 302) einer unterschiedlichen axialen Position (Z₃₀₁, Z₃₀₂) entspricht.

6. Sonde (1, 1') gemäß einem der vorangehenden Ansprüche, in der die Arme (32) regelmäßig um die Seitenwand (11) des schlauchförmigen Körpers (10) verteilt sind.

7. Sonde (1, 1') gemäß einem der vorangehenden Ansprüche, in der die Arme (32) wenigstens zum Teil in Kanälen (14) untergebracht sind, die sind in der Seitenwand (11) des schlauchförmigen Körpers (10) erstrecken.

8. Sonde (1, 1') gemäß Anspruch 7, in der die Arme (32) sich in der Seitenwand (11) des schlauchförmigen Körpers (10) in einer Richtung schräg bezüglich der Achse des Letztgenannten erstrecken.

9. Sonde (1, 1') gemäß Anspruch 7 oder 8, in der die Mittel, um die Arme aus der ersten in die zweite Position übergehen zu lassen, eine Feder (20) umfassen, die so konstruiert ist, dass sie auf die Arme (32) eine Kraft ausübt, die parallel zur Achse des schlauchförmigen Körpers (11) gerichtet ist, derart, dass sie zur Außenseite dieses Letztgenannten durch die Kanäle (14) gedrückt werden.

10. Sonde (1, 1') gemäß Anspruch 9, in der die Arme (32) flexibel sind und so konstruiert sind, dass sie durch eine Translationsbewegung von der ersten in die zweite Position übergehen.

11. Sonde (1) gemäß einem der Ansprüche 9 oder 10, in der die Mittel, um die Arme aus der zweiten Position in die erste Position übergehen zu lassen, einen Stift (50) umfassen, der durch Drücken betätigt werden kann, um eine Kraft entgegengesetzt zu der der Feder (20) auszuüben, derart, dass die Arme durch die Kanäle (14) in den schlauchförmigen Körper (11) zurückkehren gelassen werden.

12. Sonde (1) gemäß Anspruch 11, in der der Stift eine Teilspitze (50) ist, die in das Lumen (12) des schlauchförmigen Körpers (11) eingeführt werden kann, um ihn im Hinblick auf sein Einsetzen in den Körper eines Patienten zu versteifen.

13. Sonde (1') gemäß einem der Ansprüche 9 oder 10, in der die Mittel, um die Arme von der zweiten in die erste Position übergehen zu lassen, ein Kabel (52) umfassen, das durch Ziehen betätigt werden kann, um eine Kraft entgegengesetzt der der Feder (20) auszuüben, derart, dass die Arme durch die Kanäle (14) in den schlauchförmigen Körper (11) zurückkehren gelassen werden.

14. Sonde (1, 1') gemäß einem der vorangehenden Ansprüche, in der die Arme (32) aus einem elektrisch isolierenden Material hergestellt sind und jeweils wenigstens eine Elektrode (40) auf ihrer entsprechenden Oberfläche aufweisen.

15. Sonde (1, 1') gemäß Anspruch 14, in der die Arme (32), wenigstens teilweise, aus einem isolierenden biokompatiblen Material hergestellt sind, das ausgewählt ist aus Polyimiden, und insbesondere Polyisoindrochinazorindionen (PIQ) und Polyimid Pi2611, und Benzocyclobutenen.

16. Sonde gemäß einem der vorangehenden Ansprüche, in denen wenigstens einer der Arme (32) auch wenigstens eine Messelektrode (40a) umfasst, welche eine Oberfläche aufweist, die kleiner ist als die der Stimulationselektroden (40).

17. Sonde gemäß Anspruch 16, in der eine Vielzahl von Messelektroden (40a) neben jeder Stimulationselektrode angeordnet sind.

18. Sonde (1, 1') gemäß einem der vorangehenden Ansprüche, die elektrisch leitende Elemente (60) umfasst, die sich in das Lumen oder in das Innere der Seitenwand des röhrenförmigen Elements erstrecken und elektrische Kontakte mit den Elektroden (40, 40a), die von den Armen getragen werden, bilden.

19. Sonde (1, 1') gemäß Anspruch 18, in der die Elektroden (40, 40a) Planarelektroden sind, die mit den elektrisch leitenden Elementen (60) mit Hilfe von auf den Armen (32) ausgebildeten planaren Leiterbahnen (41) verbunden sind.

20. Sonde (1, 1') gemäß einem der Ansprüche 18 oder 19, in der die leitenden Elemente (60) sich bis zu einem so genannten äußeren Ende des schlauchförmigen Körpers (10) erstrecken und Mittel zum Anschluss (61) an einen Generator für elektrische Impulse zur elektrischen Tiefenneurostimulation (80) umfassen.

21. Sonde (1, 1') gemäß einem der vorangehenden Ansprüche, in der der schlauchförmige Körper (10) aus einem biokompatiblen Material hergestellt ist, das ausgewählt ist aus Silikonen, Siloxanen, Polyurethan und Polyvinylchlorid.

22. Sonde (1, 1') gemäß einem der vorangehenden Ansprüche, in der der schlauchförmige Körper (10) eine etwa zylindrische Form mit einem Durchmesser von zwischen 0,5 und 5 mm und vorzugsweise zwischen 0,5 und 2 mm aufweist.

23. Sonde (1, 1') gemäß einem der vorangehenden Ansprüche, die eine Anzahl von Armen (32) zwischen 3 und 60 und vorzugsweise zwischen 4 und 40 umfasst.

24. Sonde (1, 1') gemäß einem der vorangehenden Ansprüche, in der die Arme (32) eine Länge von zwischen 0,2 und 3 mm und vorzugsweise von zwischen 0,4 und 2 mm aufweisen.

25. Sonde gemäß einem der vorangehenden Ansprüche, die einen schlauchförmigen Körper (10) mit einer Länge von zwischen 15 cm und 40 cm hat.

26. System zur elektrischen Tiefenneurostimulation, umfassend:
- einen Generator für elektrische Impulse zur elektrischen Tiefenneurostimulation (80) und
- wenigstens eine Sonde (1) gemäß einem der vorangehenden Ansprüche, deren Stimulationselektroden elektrisch mit dem Generator für elektrische Impulse (80) verbunden sind.

27. System zur elektrischen Neurostimulation gemäß Anspruch 26, in dem wenigstens ein Arm (32) der Sonde wenigstens eine Messelektrode (40a) trägt, die eine Oberfläche aufweist, die kleiner als die der Stimulationselektroden (40) ist, wobei das System auch ein Messinstrument (80) umfasst, das elektrisch mit den Messelektroden (40a) verbunden ist.
